(19) Europäisches Patentamt / European Patent Office / Office européen des brevets

(11) **EP 1 900 353 A1**

(12) **DEMANDE DE BREVET EUROPEEN**

(43) Date de publication:
**19.03.2008   Bulletin 2008/12**

(51) Int Cl.:
*A61K 8/41* (2006.01)        *A61K 8/81* (2006.01)
*A61Q 19/00* (2006.01)        *A61Q 19/02* (2006.01)

(21) Numéro de dépôt: **07116048.5**

(22) Date de dépôt: **10.09.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **15.09.2006  FR 0653752**

(71) Demandeur: **L'ORÉAL**
**75008 Paris (FR)**

(72) Inventeur: **Baldo, Francine**
**92330, Sceaux (FR)**

(74) Mandataire: **Le Coupanec, Pascale A.M.P.**
**Nony & Associés,**
**3 rue de Penthièvre**
**75008 Paris (FR)**

(54) **Composition contre l'hyperpigmentation localisée des peaux foncées**

(57)   La présente invention concerne une composition comprenant dans un milieu physiologiquement acceptable au moins un copolymère de monomère styrénique et de diacide carboxylique à insaturation éthylénique et au moins un actif anti-séborrhéique.

EP 1 900 353 A1

**Description**

La présente invention concerne une composition cosmétique destinée à corriger ou prévenir les désordres associés à une peau grasse d'une peau foncée, notamment par une action de diminution de la sécrétion du sébum tout en conférant des propriétés dépigmentantes, comportant au moins un copolymère de monomère styrénique et de diacide carboxylique à insaturation éthylénique et au moins un actif anti-séborrhéique. L'invention a trait également à un procédé cosmétique pour corriger ou prévenir les désordres cutanés associés à une hyperséborrhée de peaux foncées. Plus particulièrement, ce procédé permet de lutter conjointement contre l'hyperséborrhée et l'hyperpigmentation localisée pouvant surgir sur le site de cicatrisation des lésions acnéiques de peaux foncées.

D'une façon générale, on peut distinguer trois types principaux de peau : les peaux sèches, les peaux grasses, les peaux mixtes.

Une peau grasse est hyperséborrhéique et caractérisée par une sécrétion et une excrétion exagérées de sébum. Classiquement, un taux de sébum supérieur à 200 $\mu$g/cm$^2$ au niveau du front est considéré comme caractéristique d'une peau grasse. Une peau grasse au sens de la présente invention est une peau d'aspect luisant, épaisse et dont les orifices folliculaires sont dilatés ou comblés de minuscules spicules cornés, voire par des comédons (ceci résulte cependant davantage d'un phénomène de rétention que d'une augmentation de l'excrétion). La peau grasse est souvent associée à un défaut de desquamation, et à un grain de peau épais. Un autre signe cutané des peaux grasses est la présence de lésions.

En plus de ces désordres esthétiques, l'excès de sébum peut servir de support au développement anarchique de la flore bactérienne saprophyte (en particulier *Propionibacterium acnes* et *Pityrosporum ovale*), et causer des comédons et/ou des lésions acnéiques.

Les lésions acnéiques se manifestent sous forme de boutons qui finissent pas se dessécher.

Dans le cas des peaux foncées, notamment de phototype IV à VI, la formation des lésions acnéiques puis des boutons évoqués ci-dessus, fait fréquemment apparaître des cicatrices qui s'accompagnent généralement d'une hyperpigmentation de la peau dans la zone de cicatrisation, accentuant encore plus le défaut cutané émanant de la lésion acnéique elle-même.

Ceci peut en particulier être observé pour les peaux foncées de type africain. Ces zones d'hyperpigmentation localisées se présentent sous forme de taches plus foncées qui sont longues ensuite à disparaître naturellement. Le processus de disparition peut prendre jusqu'à plusieurs semaines. Une solution pour traiter ces taches est d'employer un actif dépigmentant. Mais les actifs dépigmentant classiques tels que l'hydroquinone et ses dérivés, ou le résorcinol, sont des actifs trop agressifs qui ne conviennent pas pour le traitement de l'acné. En effet, ces actifs agressifs accentuent l'irritation, l'inflammation produite par l'acné et leur utilisation n'est donc pas compatible avec un traitement anti-acné.

Il existe donc un besoin de disposer d'une composition cosmétique permettant de pallier les hyperpigmentations localisées décrites ci-dessus rencontrées pour les peaux foncées tout en luttant contre la peau grasse et/ou l'hyperséborrhée et en prévenant les lésions acnéiques qui en découlent.

Le copolymère de monomère styrénique et de diacide carboxylique à insaturation éthylénique a été déjà mis en oeuvre dans des compositions cosmétiques.

En particulier, il est connu du document EP 1 374 852 de l'associer à un actif sensible à l'oxydation dans le but de stabiliser ledit actif.

Il est par ailleurs connu du document WO 2004/028483 une composition aqueuse comprenant au moins un sel métallique d'acide ascorbique phosphorylé, au moins un filtre UV hydrosoluble comportant au moins une fonction sulfonique et au moins un polymère d'anhydride maléique, les copolymères comportant des monomères anhydrides maléique et des comonomères de type styrène étant décrits.

Aucun de ces deux documents ne mentionne l'effet bénéfique de tels copolymères dans des compositions destinées à traiter les peaux grasses et/ou l'hyperséborrhée et/ou à prévenir les lésions acnéiques ainsi que les imperfections qui en découlent.

Les inventeurs ont découvert de façon surprenante que l'association d'au moins un copolymère de monomère styrénique et de diacide carboxylique à insaturation éthylénique et d'au moins un actif anti-séborrhéique présente une activité intéressante pour traiter les peaux grasses et/ou l'hyperséborrhée et pour prévenir les lésions acnéiques qui en découlent et plus particulièrement pour éviter les troubles esthétiques associés à la surproduction de sébum, en particulier les cicatrices qui apparaissent plus particulièrement dans le cas des peaux foncées. En effet, l'excès de sébum peut induire, comme exposé précédemment, le développement anarchique de certaines bactéries pathogènes telles que le *Propionibacterium acnes,* et conduire à des lésions acnéiques. Ces lésions acnéiques finissent par sécher et apparaît alors une cicatrice qui, par exemple, dans le cas des peaux foncées, peut conduire à une hyperpigmentation inesthétique sur le site de cicatrisation. L'association découverte permet conjointement de lutter contre l'hyperséborrhée et de réduire le brunissement local formé lors de la cicatrisation des lésions acnéiques.

De façon plus précise, l'invention a donc pour objet une composition comprenant, dans un milieu physiologiquement acceptable, au moins un copolymère de monomère styrénique et de diacide carboxylique à insaturation éthylénique et au moins un actif anti-séborrhéique.

L'invention a également pour objet un procédé cosmétique pour le traitement des peaux grasses et/ou de l'hyperséborrhée et pour la prévention des lésions acnéiques qui en découlent et/ou pour lutter contre les troubles esthétiques ou les imperfections associés à la surproduction de sébum de peaux foncées, notamment de phototype IV à VI, comprenant au moins une étape consistant à appliquer sur la peau une composition selon l'invention.

L'invention a aussi pour objet un procédé cosmétique pour lutter conjointement contre les peaux grasses et contre l'hyperpigmentation localisée sur les sites de cicatrisation des lésions acnéiques découlant de l'hyperséborrhée de peaux foncées, notamment de phototype IV à VI, comprenant au moins une étape consistant à appliquer sur la peau une composition selon l'invention.

L'invention a enfin pour objet l'utilisation d'une association d'au moins un copolymère de monomère styrénique et de diacide carboxylique à insaturation éthylénique et d'au moins un actif anti-séborrhéique pour la préparation d'une composition dermatologique destinée à traiter les peaux grasses et/ou l'hyperséborrhée de peaux foncées, notamment de phototype IV à VI.

En particulier, une peau foncée visée par l'invention appartient aux phototypes IV à VI de la classification de FITZPATRICK.

Au sens de la présente invention, on entend par "peaux foncées", notamment des peaux dont la clarté moyenne L* mesurée sur le front, les pommettes et le menton dans l'espace colorimétrique CIE 1976, est inférieure à 55. La saturation C* peut être comprise par exemple entre 10 et 30, notamment entre 12 et 28. Les valeurs d'angle de teinte h en ° peuvent être comprises par exemple entre 38° environ et 54° environ. Les valeurs de clarté L* peuvent être inférieures ou égales à 50, voire 45 ou 40 pour les peaux les plus sombres, tout en pouvant rester, pour la plupart des peaux, supérieures à 30.

Ces peaux peuvent encore être classées sur la base de leur réactivité aux effets du rayonnement solaire selon l'échelle proposée par FITZPATRICK.

Selon cette échelle, les différentes peaux existantes peuvent être distinguées selon les phototypes suivants :

| Type | Réactivité de la peau | Origine |
|------|----------------------|---------|
| I | Brûle toujours, ne bronze jamais | Celte |
| II | Brûle toujours, bronze peu | Germanique |
| III | Brûle modérément, bronze progressivement | Européen |
| IV | Brûle faiblement, bronze très facilement | Méditerranéen, Indien, Asiatique |
| V | Brûle rarement, bronze intensément | Moyen Orient - Sud Américain |
| VI | Ne brûle jamais, fortement pigmentée | Africain |

Les peaux foncées qui font plus particulièrement l'objet de la présente invention appartiennent aux phototypes IV à VI.

Sont plus particulièrement représentatives des phototypes IV, V et VI, des populations d'origine ethnique telle que par exemple africaine, maghrébine, indienne et afro-américaine.

## COPOLYMERE DE MONOMERE STYRENIQUE ET DE DIACIDE CARBOXYLIQUE A INSATURATION ETHYLENIQUE

Le copolymère utilisé dans la composition selon l'invention est un copolymère de monomère styrénique et de diacide carboxylique à insaturation éthylénique.

Comme monomère styrénique, on peut citer le styrène, l'alpha-méthyl styrène, et de préférence le styrène.

Comme monomère acide dicarboxylique à insaturation éthylénique, on peut citer l'acide maléique, l'acide itaconique et leurs dérivés anhydrides hydrolysés après polymérisation et notamment l'anhydride maléique et l'anhydride itaconique.

Des copolymères convenant plus particulièrement à la mise en oeuvre de l'invention sont des copolymères obtenus par copolymérisation d'une ou plusieurs unité(s) anhydride maléique ou itaconique suivi de leur hydrolyse.

Les unités anhydride maléique ou anhydride d'acide itaconique peuvent être partiellement ou totalement hydrolysées.

Ledit copolymère est de préférence sous forme de sel, notamment sous forme de sels alcalins, par exemple sous forme de sels d'ammonium, de sodium, de potassium ou de lithium, et de préférence sous forme de sel de sodium.

Dans un aspect avantageux de l'invention, le copolymère possède une fraction molaire en unité diacide carboxylique à insaturation éthylénique comprise entre 0,1 et 0,9, de préférence entre 0,4 et 0,9, et plus préférentiellement entre 0,4 et 0,6.

De préférence ledit polymère a une solubilité dans l'eau à 25 ˚C supérieure ou égale à 2 g/l.

La masse molaire moyenne en poids du copolymère styrènique/diacide carboxylique à insaturation éthylénique est de préférence comprise entre 1000 et 500 000 et de préférence entre 1000 et 50 000.

De façon préférentielle, on utilisera un copolymère de styrène et d'acide maléique (ou d'anhydride maléique hydrolysé), dans un rapport molaire styrène/acide malléique allant de 40/60 à 60/40, en particulier égal à 50/50.

Dans le cadre de la présente invention, le copolymère est avantageusement un copolymère de styrène et d'acide maléique.

On pourra utiliser par exemple, le copolymère styrène/anhydride maléique (50/50), hydrolysée sous forme de sel d'ammonium à 30 % dans l'eau vendu sous la référence SMA1000H® par la société CRAY VALLEY ou le copolymère styrène/anhydride maléique (50/50) hydrolysé sous forme de sel de sodium à 40 % dans l'eau vendu sous la référence SMA1000HNa® par la société CRAY VALLEY (et ayant pour nom INCI : sodium styrène/MA copolymer).

Certains polymères de styrène et de diacide carboxylique éthylénique présentent une bonne activité dépigmentante et antipigmentante de la peau. L'utilisation de ces polymères présente en outre l'intérêt d'être non-irritants, non-toxiques et non-allergisants pour la peau. Les exemples 1 et 2 rapportés ci-après illustrent plus particulièrement cette propriété.

Le copolymère est présent dans la composition utilisée selon l'invention en quantité suffisante pour lui conférer une propriété dépigmentante de la peau. De préférence le copolymère est présent en une teneur allant de 0,1 % à 40 % en poids, par rapport au poids total de la composition, plus particulièrement en une teneur allant de 0,1 à 20 % en poids, et préférentiellement allant de 0,1 % à 10 % en poids, par rapport au poids total de la composition.

Selon un mode particulier de réalisation de l'invention, la composition est exempte d'un ingrédient actif hydrophile sensible à l'oxydation.

Par actif hydrophile, on entend un composé ayant une solubilité dans l'eau d'au moins 0,25 % à température ambiante (25 ˚C).

Par actif hydrophile sensible à l'oxydation on entend tout actif hydrophile d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

En particulier, l'actif hydrophile sensible à l'oxydation peut être l'acide ascorbique ou l'un de ses dérivés notamment choisis parmi le 5,6-di-O-diméthylsilylascorbate, le sel de potassium du dl-alpha-tocopheryl-dl-ascorbyl-phosphate, l'ascorbyl phosphate de magnésium, l'ascorbyl phosphate de sodium, l'ascorbyl glucoside; le phloroglucinol et l'acide kojique.

## ACTIF ANTI-SEBORRHEIQUE

La composition selon la présente invention comprend au moins un actif anti-séborrhéique. On entend par actif anti-séborrhéique un composé capable de réguler l'activité des glandes sébacées.

Comme agents anti-séborrhéiques utilisables dans la composition selon l'invention, on peut citer :

- l'acide rétinoïque, le peroxyde de benzoyle, le soufre, la vitamine B6 (ou pyridoxine), le chlorure de sélénium, la criste marine ;
- les mélanges d'extrait de canelle, de thé et d'octanoylglycine tel que le Sepicontrol A5 TEA® de chez Seppic ;
- le mélange de canelle, de sarcosine et d'octanoylglycine, commercialisé notamment par la société SEPPIC sous la dénomination commerciale Sepicontrol A5®;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc;
- les dérivés de cuivre et en particulier le pidolate de cuivre tel que Cuivridone® de Solabia ;
- des extraits de végétaux des espèces *Arnica montana, Cinchona succirubra, Eugenia caryophyllata, Humulus lupulus, Hypericum perforatum, Mentha piperita, Rosmarinus officinalis, Salvia oficinalis* et *Thymus vulgaris,* tous commercialisés par exemple par la société MARUZEN;
- les extraits de reine des prés (*spiraea ulamaria*) tel que celui vendu sous la dénomination Sébonormine® par la société Silab ;
- les extraits d'algue *laminaria saccharina* tel que celui vendu sous la dénomination Phlorogine® par la société Biotechmarine ;
- les mélanges d'extraits de racines de pimprenelle (*sanguisorba officinalis/poterium officinale*), de rhizomes de gingembre (*zingiber officinalis*) et d'écorce de cannelier (*cinnamomum cassia*) tel que celui vendu sous la dénomination Sebustop® par la société Solabia ;

- les extraits de graines de lin tel que celui vendu sous la dénomination Linumine® par la société Lucas Meyer ;
- les extraits de Phellodendron tels que ceux vendu sous la dénomination Phellodendron extract BG par la société Maruzen ou Oubaku liquid B par la société Ichimaru Pharcos ;
- les mélanges d'huile d'argan, d'extrait de *serenoa serrulata* (saw palmetto) et d'extrait de graines de sésame tel que celui vendu sous la dénomination Regu SEB® par la société Pentapharm ;
- les mélanges d'extraits d'epilobe, de *terminalia chebula,* de capucine et de zinc biodisponible (microalgues) tel que celui vendu sous la denomination Seborilys® par la société green tech ;
- les extraits de *Pygeum afrianum* tel que celui vendu sous la dénomination Pygeum afrianum sterolic lipid extract par la société Euromed ;
- les extraits de *serenoa serrulata* tels que ceux vendus sous les dénomination Viapure Sabal par la société Actives International, ou ceux vendus par la société Euromed ;
- les mélanges d'extraits de plantain, de *berberis aquifolium* et de salicylate de sodium tels que celui vendu sous la dénomination Seboclear® par la société Rahn ;
- l'extrait de clou de girofle tel que celui vendu sous la dénomination Clove extract Powder par la société Maruzen ;
- l'huile d'argan telle que celle vendue sous la dénomination Lipofructyl® par les Laboratoires Sérobiologiques ;
- les filtrats de protéine lactique tels que celui vendu sous la dénomination Normaseb® par la société Sederma ;
- les extraits d'algue *laminaria,* tel que celui vendu sous la dénomination Laminarghane® par la société Biotechmarine ;
- les oligosaccharides d'algue *laminaria digitata* tel que celui vendu sous la dénomination Phycosaccharide AC par la société Codif;
- les extraits de sucre de canne tel que celui commercialisé sous la dénomination Policosanol® par la société Sabinsa ;
- l'huile de schiste sulfonée, telle que celle vendue sous la dénomination Ichtyol Pale® par la société Ichthyol ;
- les extraits d'ulmaire (*spiraea ulmaria*) tels que celui vendu sous la dénomination Cytobiol® Ulmaire par la société Libiol;
- l'acide sébacique, notamment vendu sous la forme d'un gel de polyacrylate de sodium sous la dénomination Sebosoft® par la société Sederma ;
- les glucomannanes extraits de tubercule de konjac et modifié par des chaînes alkylsulfonates tel que celui vendu sous la dénomination Biopol Beta par la société Arch Chemical ;
- les extraits de *Sophora angustifolia,* tels que ceux vendus sous la dénomination Sophora powder ou Sophora extract par la société Bioland ;
- les extraits de *cinchona succirubra* bark tel que celui vendu sous la dénomination le Red bark HS par la société Alban Muller ;
- les extraits de *quillaja saponaria* tel que celui vendu sous la dénomination Panama wood HS par la société Alban Muller ;
- la glycine greffée sur chaîne undécylénique, telle que celle vendue sous la dénomination Lipacide UG OR par la société Seppic ;
- le mélange d'acide oléanolique et d'acide nordihydroguaïarétique, tel que celui vendus sous la forme d'un gel sous la dénomination AC.Net par la société Sederma ;
- l'acide phthalimidoperoxyhexanoïque ;
- le citrate de trialkyle($C_{12}$-$C_{13}$) vendu sous la dénomination COSMACOL® ECI par la société Sasol ; le citrate de trialkyle($C_{14}$-$C_{15}$) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ; et
- leurs mélanges.

Comme actif anti-séborrhéique préférés, on peut citer :

- le peroxyde de benzoyle, la vitamine B6 (ou pyridoxine),
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc;
- les extraits de reine des prés (*spiraea ulamaria*) tel que celui vendu sous la dénomination Sébonormine® par la société Silab ;
- les extraits d'algue *laminaria saccharina* tel que celui vendu sous la dénomination Phlorogine® par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- l'extrait de clou de girofle tel que celui vendu sous la dénomination Clove extract Powder par la société Maruzen ;
- les filtrats de protéine lactique tels que celui vendu sous la dénomination Normaseb® par la société Sederma ;

- les extraits d'ulmaire (*spiraea ulmaria*) tels que celui vendu sous la dénomination Cytobiol® Ulmaire par la société Libiol ;
- l'acide sébacique, notamment vendu sous la forme d'un gel de polyacrylate de sodium sous la dénomination Sebosoft® par la société Sederma ;
- la glycine greffée sur chaîne undécylénique, telle que celle vendue sous la dénomination Lipacide UG OR par la société Seppic ;
- le citrate de trialkyle($C_{12}$-$C_{13}$) vendu sous la dénomination COSMACOL® ECI par la société Sasol; le citrate de trialkyle($C_{14}$-$C_{15}$) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience;
- et leurs mélanges.

Préférentiellement, l'actif anti-séborrhéique est choisi parmi :

- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc; et de préférence le pyrrolidone carboxylate de zinc (ou pidolate de zinc) ou le salicylate de zinc;
- l'extrait de clou de girofle tel que celui vendu sous la dénomination Clove extract Powder par la société Maruzen ;
- la glycine greffée sur chaîne undécylénique, telle que celle vendue sous la dénomination Lipacide UG OR par la société Seppic ;
- le citrate de trialkyle($C_{12}$-$C_{13}$) vendu sous la dénomination COSMACOL® ECI par la société Sasol ; le citrate de trialkyle($C_{14}$-$C_{15}$) vendu sous la dénomination COSMACOL® ECL par la société Sasol ;
- et leurs mélanges.

L'actif anti-séborrhéique est par exemple présent dans une teneur allant de 0,1 à 10 % en poids, de préférence de 0,1 à 5 % en poids, et préférentiellement de 0,5 % à 3 % en poids, par rapport au poids total de la composition.

## ACTIF ANTI-ACNE

Dans un aspect avantageux de l'invention, la composition peut comporter en outre au moins un actif anti-acné.
Par "actif anti-acné", on entend notamment tout actif ayant des effets sur la flore spécifique des peaux grasses tels que par exemple le Propionibacterium acnes (*P acnes*). Ces effets peuvent être bactéricides.
A titre d'actifs antibactéricide, on peut citer notamment :

- les actifs et conservateurs à activité anti-microbienne cités dans la demande DE10324567, incorporée dans la présente invention par référence,
- l'acide asiatique,
- le sel de monoéthanolamine du 1-hydroxy-4-methyl 6-trimethylpentyl 2-pyridone (nom INCI: piroctone olamine), notamment vendu sous la dénomination Octopirox® par la société Clariant ;
- l'acide citronellique, l'acide périllique (ou acide 4-isopropenylcyclohex-1-ènecarboxylique),
- le 2-éthyl hexyle éther de glycérol (nom INCI : ethylhexylglycerine), par exemple vendu sous la dénomination Sensiva SC 50® par la société Shulke & Mayr,
- le caprylate/caprate de glyceryle, par exemple vendu sous la dénomination Capmul MCM® par la société ABITEC;
- le phosphosilicate de calcium et de sodium, notamment vendu sous les dénominations Bioactive glasspowder® et Actysse Premier BG® par la société Schott Glass ;
- les particules à base d'argent, par exemple celle vendues sous la dénomination Métashine ME 2025 PS® par la société Nippon Sheet Glass ;
- l'extrait de cône de houblon (Humulus Lupulus) obtenu par extraction $CO_2$ supercritique tel que celui vendu sous la dénomination HOP CO2-TO extract® par la société Flavex Naturextrakte,
- l'extrait de Millepertius obtenu par extraction $CO_2$ supercritique tel que celui vendu sous la dénomination ST John's Wort CO2-TO extract® par la société Flavex Naturextrakte,
- le mélange d'extraits de racines de *scutellaria baicalensis,* de *paeonia suffruticosa* et de *glycyrrhiza glabra,* tel que celui vendu sous la dénomination BMB - CF® par la société Naturogin,
- l'extrait d'arganier comme l'Argapure LS9710® de chez COGNIS ;
- les extraits de feuilles de busserole comme celui vendu sous la dénomination "Melfade-J" par la société Pentapharm ;
- l'acide 10-hydroxy-2 décanoique tel que l'Acnacidol P® de chez Vincience, l'ursolate de sodium, l'acide azélaïque, le di-iodo-méthyl p-tolylsulfone tel que l'Amical Flowable® de chez Angus, la poudre de malachite, l'oxyde de zinc tel que Zincare® de chez Elementis GMBH, l'acide octadécènedioïque tel que l'Arlatone dioic DCA® de chez Uniqema ; l'acide ellagique ; le 2,4,4'-trichloro-2'-hydroxy diphényl éther (ou triclosan), la 1-(3',4'-dichlorophényl)-3-(4'-chlorophényl)urée (ou triclocarban), le 3,4,4'-trichlorocarbanilide, le 3',4',5'-trichlorosalicylanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopirox, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octoxyglycérine ou octoglycérine, l'octanoylglycine tel que Lipacid C8G® de Seppic, le caprylyl glycol, l'acide 10-hydroxy-2-décanoïque, le dichlorophenyl imidazol dioxolan et ses dérivés décrits dans la demande de brevet WO9318743, l'iodopropynyl butylcarbamate, le 3,7,11-triméthyldodéca-2,5,10-triénol ou farnesol, les phytosphingosines ; les sels d'ammonium quaternaires comme les sels de cetyltrimethylammonium, les sels de cétylpyridinium, et

- leurs mélanges.

On peut également citer certains tensioactifs ayant un effet antimicrobien comme le cocoampho acétate de sodium ou diacetate disodium tel que le Miranol C2M CONC NP, les bétaines comme la cocoyl bétaine Genagen KB de Clariant, le lauryl ether sulfate de sodium comme l'Emal 270 D de Kao, le décyl glucoside comme le Plantacare 2000 UP, les malate de dialcools $C_{12-13}$ ramifiés comme le Cosmacol EMI, les monoesters de propylène glycol comme monolaurate, monocaprylate, monocaprate de propylène glycol, le lauryl dimethylamine betaine comme le Empigen BB/LS ainsi que les polyammonium quaternaires comme le Quaternium-24 ou Bardac 2050 de Lonza et ceux décrits dans le brevet FR 0 108 283 et leurs mélanges.

Comme agents antimicrobiens préférés, on utilisera dans les compositions de l'invention un agent choisi parmi le caprylyl glycol, l'octoglycérine ou octoxyglycérine, l'acide 10-hydroxy-2-décanoïque, et leurs mélanges.

D'autres actifs anti-acné additionnels peuvent être ajoutés aux actifs anti-acnés précités. On peut notamment citer les actifs présentant des effets anti-adhésion bactérienne ou bien agissant sur le biofilm des bactéries pour éviter leur multiplication.

Comme agents prévenant et/ou réduisant l'adhésion des micro-organismes, on peut citer notamment : le phytanetriol et ses dérivés tels que décrits dans la demande de brevet EP 1 529 523, les huiles végétales telles de l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia, décrites dans le brevet EP 1 133 979, ou encore certains tensioactifs tels que le cocoamphodiacétate disodique, le cocoate de glycéryle oxyéthyléné (7 OE), fhexadécénylsuccinate 18, le palmitate d'octoxyglycéryle, le béhénate d'octoxyglycéryle, l'adipate de dioctyle, le PPG-15 stéaryl éther, le tartrate de di-alcools $C_{12}$-$C_{13}$ ramifiés décrits dans le brevet EP 1 129 694 et leurs mélanges.

En particulier vis-à-vis de la propagation du *P acnes,* ou en tant qu'actifs agissant sur le biofilm des bactéries pour éviter leur prolifération, on peut citer le pentylène glycol, le nylon-66 (fibres de polyamides 66), l'huile de son de riz , l'alcool polyvinylique tel que Celvol 540 PV alcohol® de chez Celanese Chemical, l'huile de colza telle que Akorex L® de chez Karlshamns, et les dérivés de fructose et leurs mélanges.

L'actif agissant contre l'acné peut être présent dans une teneur allant de 0,01 à 10 % en poids, de préférence de 0,05 à 5 % en poids par rapport au poids total de la composition.

## AGENT DESQUAMANT

Dans un autre aspect avantageux de l'invention, les compositions selon la présente invention peuvent comprendre en outre un agent desquamant.

Par "agent desquamant", on entend tout composé capable d'agir :

- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'acide 8-hexadécène 1,16-dicarboxylique ; l'acide gentisique et ses dérivés ; les oligofucoses ; l'acide cinnamique ; l'extrait de Saphora japonica ; le resvératrol et certains dérivés d'acide jasmonique ;

- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques tels que décrits dans la demande WO 02/39975 et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; l'O-octanoyl-6-D-maltose et la N-acétyl glucosamine.

Les mélanges d'agents desquamants précités sont également compris à titre d'agents desquamants.

Comme autres agents desquamants utilisables dans la composition selon l'invention, on peut citer :

- les oligofructoses, l'EDTA et ses dérivés, les laminaria extract, le o-linoleyl-6D-glucose, l'acide (3-hydroxy-2pentylcyclopentyl)-acétique, le trilactate de glycérol, l'O-octanyl-6'-D-maltose, la S carboxyméthyl cystéine, les dérivés siliciés de salicylate comme ceux décrits dans le brevet EP 0 796 861, les oligofucases comme ceux décrits dans le brevet EP 0 218 200, l'acide jasmonique et dérivés comme décrits dans les demandes de brevet EP 1 333 022 et EP 1 333 021,
- l'extrait de fleur de ficus *opuntia indica* (Exfolactive® de Silab),
- leurs mélanges.

Comme agents desquamants préférés, on pourra citer les β-hydroxyacides, tel que l'acide n-octanoyl 5-salicylique; les acides glycolique, citrique, lactique, tartrique, malique ou mandélique ; l'acide (N-2 hydroxyéthylpiperazine-N-2-éthane) sulfonique (HEPES) ; l'extrait de Saphora japonica ; le miel ; le N-acétyl glucosamine ; le méthyl glycine diacétate de sodium, et leurs mélanges.

Encore plus préférentiellement on utilisera dans les compositions de l'invention un agent desquamant choisi parmi l'acide n-octanoyl 5-salicylique ; l'urée; l'acide (N-2 hydroxyéthylpiperazine-N-2-éthane) sulfonique (HEPES); l'extrait de Saphora japonica ; le miel ; le N-acétyl glucosamine; le méthyl glycine diacétate de sodium, et leurs mélanges.

## AUTRES AGENTS

Selon encore un autre aspect avantageux de l'invention, les compositions selon la présente invention peuvent en outre comprendre au moins un agent apaisant et/ou au moins une actif astringent et/ou au moins un actif matifiant et/ou au moins un actif cicatrisant et/ou au moins un actif agissant sur la prolifération kératinocytaire.

On entend par agent apaisant un composé permettant de réduire la sensation de picotements, de démangeaisons ou de tiraillements de la peau.

Comme agents apaisants utilisables dans la composition selon l'invention, on peut citer : les oligomères procyannidoliques, les vitamines E, C B5, B3, la caféine et ses dérivés, les triterpènes pentacycliques et les extraits de plantes les contenant, l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra), l'acide oléanolique et ses sels, l'acide ursolique et ses sels, l'acide boswellique et ses sels, l'acide bétulinique et ses sels, un extrait de *Paeonia suffruticosa* et/ou *lactiflora,* un extrait de *Laminaria saccharina,* les extraits de *Centella asiatica,* l'huile de Canola, le bisabolol, le diesterphosphorique de vitamine E et C comme le Sepivital EPC® de Seppic, les extraits de camomille, l'allantoïne, les huiles insaturées en oméga 3 telles que les huiles de rosier musca, de cassis, d'Ecchium, de poisson, l'huile de calophilum, des extraits de plancton, la capryloyl glycine, un mélange d'extrait de fleur de nénuphar et de palmitoylproline tel que celui vendu sous la dénomination "Seppicalm VG®" par la société Seppic, un extrait de *Boswellia serrata,* un extrait de *Centipeda cunnighami* tel que celui vendu sous la dénomination "Cehami PF®" par la société TRI-K Industries, un extrait de graines de tournesol en particulier l'Hélioxine® de Silab, un extrait de graines de *Linum usitatissimum* comme la Sensiline® de Silab, les tocotriénols, le piperonal, un extrait *d'Epilobium angustifolium* tel que celui vendu sous la dénomination "Canadian Willowherb Extract" par la société FYTOKEM PRODUCTS, l'aloe vera, les phytostérols, l'eau de bleuet, l'eau de rose, un extrait de menthe, en particulier de feuilles de menthe comme le Calmiskin® de Silab, les dérivés d'anis, les bactéries filamenteuse comme Vitreoscilla filiformis tel que décrit dans le brevet EP 761 204, un extrait de pétales de rose comme le Rose Flower Herbasol® extract de la société Cosmetochem, le beurre de karité, un mélange de fraction de cire de graine d'orge obtenue par $CO_2$ supercritique, de beurre de karité et d'huile d'argan comme le "Stimu-tex AS®" de Pentapharm, les sels alcalino-terreux notamment le strontium, et leurs mélanges.

Comme agents apaisants préférés selon l'invention, on utilisera :

l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra) ; l'acide ursolique et ses sels; les extraits de Centella asiatica, l'huile de Canola, le bisabolol ; les extraits de camomille, l'allantoïne ; un mélange d'extrait de fleur de nénuphar et de palmitoylproline tel que celui vendu sous la dénomination "Seppicalm VG®" par la société Seppic ; l'aloe vera, l'eau de rose, un extrait de menthe, en particulier de feuilles de menthe comme le Calmiskin® de Silab, les bactéries filamenteuse comme Vitreoscilla filiformis tel que décrit dans le brevet EP 761 204, un extrait de pétales de rose comme le Rose Flower Herbasol® extract de la société Cosmetochem, le beurre de karité, et leurs mélanges.

Préférentiellement, l'agent apaisant est choisi parmi :

- le bisabolol ; un mélange d'extrait de fleur de nénuphar et de palmitoylproline tel que celui vendu sous la dénomination "Seppicalm VG®" par la société Seppic ; l'aloe vera, l'eau de rose, un extrait de menthe, en particulier de feuilles de menthe comme le Calmiskine® de Silab, les bactéries filamenteuse comme Vitreoscilla filiformis tel que décrit dans le brevet EP 761 204, un extrait de pétales de rose comme le Rose Flower Herbasol® extract de la société Cosmetochem, le beurre de karité, et leurs mélanges.

Par "agents astringents", on entend selon l'invention des agents permettant de lutter contre la dilatation des follicules sébacés.

Comme agents astringents utilisables dans la composition selon l'invention, on peut citer des extraits de pulpe de champignon (*polyporus officinalis*) comme le "Laricyl LS8865®" de Cognis, des extraits de *Terminalia catappa* et *sambucus nigra* comme le Phytofirm LS9120® de Cognis, des extraits de noix de galle comme le Tanlex VE® de Ichimaru Pharcos, l'hydroxychlorure d'aluminium, les extraits de centella (ex Plantactiv centella de Cognis), le chlorure de dicétyl diméthyl ammonium comme le Varisoft 432 CG® de Degussa, les extraits de marron d'inde, les extraits de mauve, les extraits d'Hammamelis, des extraits d'amandes douces, de racines de guimauve et de graines de lin comme l'Almondermin LS 3380® de Cognis, les extraits de bardane, les extraits d'ortie, les extraits de bouleau, les extraits de prêle, les extraits de camomille comme ceux vendus sous la dénomination Extrapone 9 special® par la société Symrise, les extraits de scutellaria, les extrais d'Ulmaire (par exemple le Cytobiol Ulmaire de Libiol), un mélange d'extraits de gingembre blanc, de prêle, d'ortie, de romarin, de yucca comme l'Herb extract B1348® de Bell flavors & fragrances, les extraits d'acacia, d'orme, de saule blanc, de canelle, de bouleau, de reine des prés, les sapogénines de panama, le phenolsulfonate de zinc de Interchemical, des extraits de gentiane, de concombre, de noyer, le mélange d'extraits de Ratanhia, de pamplemousse, de grindelia et de galle de chêne comme l'Epilami® de Alban Muller.

Comme agents astringents préférés selon l'invention, on utilisera les extraits de scutellaria, les extraits d'ulmaire, les extraits de reine des près, les extraits de gentiane, les extraits de bardane et leurs mélanges.

Comme exemples d'agents cicatrisants, on peut citer notamment:

- l'allantoine, l'urée, certains acides aminés comme l'hydroxyproline, l'arginine, la sérine, et aussi des extraits de lys blanc (comme le Phytélène Lys 37EG 16295 de Indena), un extrait de levures comme le cicatrisant LS LO/7225B des Laboratoires Sériobiologiques), l'huile de tamanu, l'extrait de saccharomyces cerevisiae comme le Biodynes® TRF® de Arch Chemical, les extraits d'avoine, le chitosane et dérivés comme le glutamate de chitosane, les extraits de carotte, l'extrait d'artemia comme le GP4G® de Vincience, l'acexamate de sodium, des extraits de lavandin, des extraits de propolis, l'acide ximeninique et ses sels, l'huile de rosa rugosa, des extraits de souci comme le Souci Ami® Liposoluble d'Alban Muller, des extraits de prêle, les extraits d'écorce de citron comme l'Herbasol® citron de Cosmetochem, des extraits d'helichryse, des extraits de millefeuilles.

Comme agents cicatrisants préférés selon l'invention, on utilisera l'huile de tamanu, l'acexamate de sodium, des extraits de prêle, des extraits d'helichryse, et leurs mélanges.

Par "agent matifiant", on entend des agents destinés à rendre la peau visiblement plus mate, moins brillante.

L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

L'agent matifiant pourra notamment être choisi parmi un amidon de riz ou un amidon de maïs, la kaolinite, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de polymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les particules de silicate et notamment de silicate mixte, et leurs mélanges.

Comme exemples d'agents matifiants, on peut citer notamment :

- l'amidon de riz ou de maïs, en particulier un aluminium starch octenyl succinate commercialisé sous la dénomination Dry Flo® par la société National Starch,
- la kaolinite ;
- les silices ;
- le talc ;
- un extrait de graines de potiron tel que commercialisé sous la dénomination Curbilene® par la société Indena ;
- des microbilles de cellulose telles que décrites dans la demande de brevet EP 1 562 562 ;

- des fibres, telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742 ;
- des microsphères de copolymères acryliques expansées telles que celles commercialisées par la société EXPANCEL sous les dénominations EXPANCEL 551®,
- des charges à effet optique telles que décrites dans la demande de brevet FR 2 869 796, en particulier :

    - les poudres de polyamides (Nylon®), comme par exemple les particules de Nylon 12 du type Orgasol d'Arkema de taille moyenne 10 microns et d'indice de réfraction 1,54,
    - les poudres de silice, comme par exemple les Silica beads SB150 de Miyoshi de taille moyenne 5 microns et d'indice de réfraction 1,45,
    - les poudres de polytétrafluoroéthylène, comme les PTFE ceridust 9205F de Clariant de taille moyenne 8 microns et d'indice de réfraction 1,36,
    - les poudres de résine de silicone comme les Silicon resin Tospearl 145A de GE Silicone de taille moyenne 4,5 microns et d'indice de réfraction 1,41,
    - les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100® et F 80 ED® de la société Matsumoto Yushi-Seiyaku,
    - les poudres de cire comme les particules Paraffin wax microease 1145 de micropowders de taille moyenne 7 microns et d'indice de réfraction 1,54,
    - les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
    - les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations "KSP-100", "KSP-101", "KSP-102", "KSP-103", "KSP-104", "KSP-105" par la société SHIN ETSU, et
    - les poudres composites de talc/dioxyde de titane/alumine/silice comme celles vendues sous la dénomination Coverleaf® AR-80 par la société Catalyst & chemicals,

- leurs mélanges,
- des composés absorbant et/ou adsorbant le sébum tels que décrits dans la demande de brevet FR 2 869 796. On peut citer notamment :

    - les poudres de silice, comme par exemple les microsphères de silice poreuses vendues sous la dénomination "SILICA BEADS SB-700" commercialisées par la société MYOSHI, les "SUNSPHERE® H51", "SUNSPHERE® H33" , "SUNSPHERE® H53" commercialisées par la société ASAHI GLASS; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ;
    - les poudres de silicates mixtes amorphes, notamment d'aluminium et de magnésium, comme par exemple celle commercialisée sous la dénomination "NEUSILIN UFL2" par la société Sumitomo.
    - les poudres de polyamides (nylon®), comme par exemple "l'ORGASOL® 4000" commercialisé par la société Arkema, et
    - les poudres de polymères acryliques, notamment de polyméthacrylate de méthyle, comme par exemple le "COVABEAD® LH85" commercialisé par la société WACKHERR ; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL; de copolymère diméthacrylate d'éthylène glycol/méthacrylate de lauryle, comme par exemple le "POLYTRAP® 6603" commercialisé de la société DOW CORNING ;

- les particules de silicate, telle que la silicate d'alumine ;
- les particules de silicates mixtes, telles que :

- les particules de silicate d'aluminium et de magnésium, telles que la saponite ou silicate de magnésium et d'aluminium hydraté avec un sulfate de sodium commercialisée sous la dénomination commerciale Sumecton® par la société Kunimine ;
- le complexe silicate de magnésium, hydroxyéthylcellulose, huile de cumin noir, huile de courge et phospholipides ou Matipure® de Lucas Meyer, et
- leurs mélanges.

Comme agents matifiants préférés, on pourra utiliser selon l'invention un extrait de graines de potiron, un amidon de riz ou de mais, la kaolinite, des silices, le talc, les poudres de polyamides, les poudres de polyethylènes, les poudres de copolymères acryliques, les microsphères de copolymères acryliques expansées, les microbilles de résines de silicones, les particules de silicate mixte et leurs mélanges.

Comme actif agissant sur la prolifération kératinocytaire utilisables dans la composition selon la présente invention, on peut citer l'extrait de *Larrea divaricata* tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, le phloroglucinol, les extraits de tourteaux de noix commercialisés par Gattefosse et les extraits de *solanum tuberosum* tel que le Dermolectine® commercialisé par Sederma.

La composition selon la présente invention est adaptée à une application topique sur la peau. Le milieu physiologiquement acceptable est préférentiellement un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

Par milieu physiologiquement acceptable, on comprend un milieu compatible avec les matières kératiniques d'êtres humains comme la peau, les muqueuses, les ongles, le cuir chevelu et/ou les cheveux.

La composition selon l'invention peut être destinée à une application cosmétique ou pharmaceutique, particulièrement dermatologique. De préférence la composition selon l'invention est destinée à une application cosmétique.

La composition peut alors comprendre tous les constituants usuellement employés dans l'application envisagée.

On peut notamment citer l'eau, les solvants, les huiles d'origine minérale, animale et/ou végétale, les cires, les pigments, les charges, les tensioactifs, les actifs cosmétiques ou dermatologiques, les filtres UV, les polymères, les gélifiants, les conservateurs.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition selon l'invention peut se présenter sous toutes les formes galéniques normalement utilisées dans les domaines cosmétique et dermatologique; elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique, ou bien encore d'une poudre.

Lorsque la composition est une émulsion, la proportion de la phase grasse peut aller de 5 à 80 % en poids, et de préférence de 5 à 50 % en poids par rapport au poids total de la composition. Les huiles, les émulsionnants et les éventuels coémulsionnants utilisés dans la composition sous forme d'émulsion sont choisis parmi ceux classiquement utilisés dans le domaine considéré. L'émulsionnant et le coémulsionnant sont présents, dans la composition, en une proportion pouvant aller de 0,3 à 30 % en poids, et de préférence de 0,5 à 20 % en poids par rapport au poids total de la composition.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick.

Les exemples qui suivent illustrent la présente invention.

**Exemple 1** :

Mesure de l'effet modulateur de la mélanogénèse du copolymère styrène/anhydride maléique (50/50) hydrolysé sous forme de sel de sodium à 40 % dans l'eau (SMA1000HNa® par la société CRAY VALLEY), en présence ou en l'absence d'acide ascorbique, sur co-culture de kératinocytes et mélanocytes humains normaux

1.1. MATERIEL ET METHODE

L'effet modulateur sur la mélanogénèse du copolymère SMA1000HNa® été testé selon la méthode décrite dans le brevet FR-A-2 734 825, ainsi que dans l'article de R. Schmidt, P. Krien et M. Régnier, Anal. Biochem., 235(2), 113-18, 1996.

En bref, la méthode comprend les étapes consistant à :

mettre en co-culture des kératinocytes / mélanocytes humains normaux dans un milieu contenant l'extrait testé ainsi qu'un précurseur de mélanine marqué, en l'occurrence du thiouracile marqué au C 14,
à lyser les cellules contenant la mélanine avec des enzymes dégradant les protéines,
à faire passer l'extrait obtenu sur un filtre échangeur d'anions ayant un diamètre de pores inférieur ou égal à 1 $\mu$m, et
à évaluer la quantité de mélanine fixée par le filtre au moyen d'un dosage radioactif du thiouracile.

Précisément, 50 000 kératinocytes humains normaux et 17 000 mélanocytes humains normaux sont mélangés et ensemencés par puits de plaques de 96 puits (type Costar) et cultivés trois jours dans le milieu de différenciation. Pendant les trois jours suivants, le milieu de culture est remplacé quotidiennement par le milieu test défini (contenant les produits à évaluer, ainsi que 3 $\mu$Ci/ml de thiouracile marquée au C14).

Les témoins suivants sont réalisés :

- culture témoin : milieu test
- témoin positif de stimulation de la mélanogénèse : milieu test + 1 mM tyrosine;
- 3 témoins positifs d'inhibition de la mélanogénèse : milieu test + respectivement 500 $\mu$M d'acide kojique ; 100 $\mu$M d'arbutine ; 10 %M de lucinol (2-butyl benzène 1,3-diol).

La radioactivité totale incorporée dans les protéines est estimée par l'incorporation de leucine tritiée, prise comme indicateur de prolifération et indicateur précoce de cytotoxicité. La veille de la prise, 1 $\mu$Ci/ml de leucine tritiée est ajouté au milieu test. On considère qu'un produit est cytotoxique lorsque la teneur en leucine tritiée diminue d'au moins 30 %.

Après une nuit, les cellules sont rincées en tampon phosphate (Phosphate Buffered Saline, PBS). Les protéines sont précipitées à l'aide d'acide trichloracétique (TCA) à 5 % et lavées afin d'éliminer la radioactivité libre. Les protéines sont incubées pendant la nuit à 37 ˚C à l'aide d'une solution de protéinase K à 20 $\mu$l/ml dans du tampon Tris HCl-triton-EDTA.

50 $\mu$l d'extrait total sont prélevés et transférés dans une plaque 96 puits (Wallac) et 150 $\mu$l de liquide scintillant (Optiphase "Supermix") sont ajoutés. Le reste de l'extrait, soit 950 $\mu$l, est filtré sur filtre DEAE Filtermat. Après rinçage, le filtre recouvert du scintillant solide "Meltilex" est transféré dans une plaque de comptage. La radioactivité est comptée à l'aide du compteur Wallac. Les résultats sont exprimés en pourcentage du témoin selon la formule:

$$\frac{(14CP / 3HP) - (14CT / 3HT)}{(14CT / 3HT)} \times 100$$

dans laquelle:

14CP est la moyenne des désintégrations par minute (dpm) thiouracile 14C sur 3 puits similaires traités par un produit (P) ;
3HP est la moyenne des dpm leucine 3H correspondant ;
14CT est la moyenne des dpm thiouracile 14 C sur 3 puits similaires témoin (T);
3HT est la moyenne des dpm leucine 3H correspondant.

On calcule le rapport de l'incorporation de thiouracile à l'incorporation de leucine, pour normaliser la mélanogénèse à la quantité de protéines présente dans le puit. Ce rapport traduit la modulation de la mélanogénèse.

1.2. RESULTATS

| Références | $^3$H Leu. (%/Témoin) | $^{14}$C ThioU. (%/Témoin) | $\frac{^{14}C\ ThioU.}{^3H\ Leu.}$ |
|---|---|---|---|
| Tyrosine 1 mM | +6 % | 96 % | 85 % |
| Acide kojique 500 $\mu$M | -8% | -56 % | -52 % |
| Arbutine 100$\mu$M | -8 % | -49 % | -44 % |
| Lucinol 10$\mu$M | 4% | -60 % | -62 % |

| [vitamine C] (mM | 0,1 | 0,3 | 0,4 | 0,6 | | IC50 |
|---|---|---|---|---|---|---|
| $^3$H Leu. (%/Témoin) | 0% | -17% | -23% | -23% | | |
| $^{14}$C ThioU. (%/Témoin) | -14% | -69% | -79% | -76% | | |
| $\frac{^{14}C\ ThioU.}{^3H\ Leu.}$ | -14 % | -62 % | -72 % | -68 % | | 0,23 mM vitamine C |

| [vitamine C] (mM) | 0,1 | 0,3 | 0,4 | 0,6 | | |
|---|---|---|---|---|---|---|
| [copolymère SMA] ($\mu$M) | 18 | 53 | 70 | 105 | | IC50 |
| $^3$H Leu. (%/Témoin) | -4% | -15 % | -12% | -11% | | |
| $^{14}$C ThioU. (%/Témoin) | -28 % | -66 % | -67% | -71 % | | |
| $\frac{^{14}C\ ThioU.}{^3H\ Leu.}$ | -25 % | -60 % | -63 % | -68 % | | 0,23 mM vitamine C + 42 $\mu$M SMA |

| [copolymère SMA] ($\mu$M) | 18 | 53 | 70 | 105 | | IC50 |
|---|---|---|---|---|---|---|
| $^3$HLeu. (%/Témoin) | 2% | 4% | 0% | -16% | | |
| $^{14}$C ThioU. (%/Témoin) | 21% | -42% | -56 % | -62% | | |
| $\frac{^{14}H\ ThioU.}{^3H\ Leu.}$ | -23% | -45% | -56 % | -54% | | 60 $\mu$M SMA |

Dans les tableau, SMA signifie le copolymère SMA1000HNa®

1.3. CONCLUSIONS

Aucune cyto-toxicité des produits testés n'a été observée aux doses évaluées. La vitamine C présente une activité dépigmentante et permet d'atteindre l'IC50 sur ce modèle à environ 0.23 mM. L'activité de la vitamine C n'est pas modifiée en présence du copolymère SMA1000HNa®. En revanche, le copolymère SMA1000HNa® testé seul, dans la même gamme de concentration qu'en présence de vitamine C, présente également un potentiel dépigmentant avec effet dose. Il permet d'atteindre l'IC50 à 60 µM de copolymère. Le copolymère SMA1000HNa® a donc une activité dépigmentante qui lui est propre.

Les activités de la vitamine C et du copolymère SMA1000HNa® ne sont pas synergiques. L'activité du copolymère SMA1000HNa® semble au contraire être masquée en présence de vitamine C.

**Exemple 2 :**

2) Principe du protocole anti-pigmentation :

Mesure de l'effet modulateur de la mélanogénèse photoinduite du polymère SMA1000HNa® en présence ou en l'absence d'acide ascorbique sur co-culture de kératinocytes et mélanocytes humains normaux (effet anti-pigmentation)

2.1. MATERIEL ET METHODE

Des co-cultures Mélanocytes / Kératinocytes humains normaux (NHM/NHK) subissent 1 irradiation au rayonnement UV, répétée quotidiennement pendant 4 jours, en présence ou en absence des produits à évaluer. La synthèse de mélanine est quantifiée comme précédemment grâce à l'incorporation de [14C]thiouracile présente dans le milieu de culture. Parallèlement, un dosage protéique est effectué dans chaque puits afin de normaliser la synthèse mélanique et d'estimer la toxicité du produit. Une atténuation de l'induction de la mélanogenèse après irradiation UV peut ainsi être mise en évidence.

Plus précisément, les Mélanocytes et Kératinocytes sont ensemencés respectivement à 80000 et 250000 cellules par puits en plaque 24 puits dans le milieu de différenciation kératinocytaire. Les plaques sont incubées 3 jours à 37 ˚C avant traitement.

Pour éviter la photo oxydation d'un de ses composants, le milieu de culture est remplacé au moment de l'irradiation, par une solution des produits dans du PBS. Les produits sont mis en contact avec les cellules 15 minutes avant l'irradiation.

L'irradiation UV est réalisée à l'aide d'un simulateur solaire. La dose délivrée est de 7 J/cm$^2$. Source d'irradiation : simulateur Oriel Xénon 1000W + filtre dichroïque + WG 320/1.5 mm.

Les témoins suivants sont réalisés:

Témoin non irradiée (quantification de la mélanogenèse constitutive)
Témoin contrôle d'irradiation
2 Témoins positifs d'inhibition de la mélanogénèse photoinduite : (100 µM Arbutine ; 1 µM Lucinol)

Après irradiation, le PBS est éliminé et les cellules sont mises à incuber dans un milieu de culture semi défini auquel sont ajoutés les produits et la 14C-thiouracile (1µCi/ml). Ce traitement est renouvelé quotidiennement pendant 4 jours.

Pour chaque condition expérimentale, des lysats cellulaires sont réalisés en rinçant les cellules avec un tampon phosphate (PBS). Les protéines sont précipitées à l'aide d'acide trichloracétique (TCA) à 5 % et lavées afin d'éliminer la radioactivité libre. Les protéines sont incubées pendant un week end à 37 ˚C à l'aide d'une solution de protéinase K à 20 µl/ml dans du tampon Tris HCl-triton-EDTA.

5 µl d'extrait total sont prélevés et transférés dans une plaque 96 puits (Wallac). Les protéines sont dosées à l'aide du kit de dosage Protein Assay Reagent (Pierce). La quantité de protéines par puits (P) servira à normaliser la mélanogenèse. Le reste de l'extrait est filtré sur filtre DEAE Filtermat (Wallac). Après rinçage, le filtre recouvert du scintillant solide "Meltilex" (Wallac) est transféré dans une plaque de comptage costar. La radioactivité est comptée à l'aide du compteur Wallac et permet la quantification de la mélanogenèse (M) par mesure du [14C]C-thiouracile (ccpm) incorporé par les cellules.

$$\text{Mélanogenèse normalisée (MN)} = M / P$$

$$\text{Mélanogenèse Photo Induite (MPI)} = \text{MN (échantillon irradié)} - \text{MN (échantillon non irradié)}$$

Effet Anti-pigmentant du Produit (%) = [[MPI (échantillon irradié+traité)-MPI (échantillon irradié)] / MPI (échantillon irradié)] X 100

2-2 RESULTATS

| Références | Protéines (%/Témoin non irradié) | MPI (%/Témoin non irradié) | Effet anti-pigmentant du produit |
|---|---|---|---|
| Témoin SSR | -21 % | 225 % | - |
| Arbutine 100$\mu$M + SSR | -15 % | -24 % | -111 % |
| Lucinol 1$\mu$M + SSR | -14 % | -36 % | - 116 % |

| [vitamine C] (mM) | 0,3 | 1 | 2 | 3 | | IC50 |
|---|---|---|---|---|---|---|
| Protéines (%/Témoin non irradié) | -12 % | -19 % | -19 % | -22 % | | |
| MPI (%/Témoin non irradié) | 134% | 78% | 23% | 0 % | | |
| Effet anti-pigmentant du produit | -40% | -65% | -90% | -100% | | 0,47 mM vitamine C |

| [vitamine C] (mM) | 0,3 | 1 | 2 | 3 | | |
|---|---|---|---|---|---|---|
| [SMA] ($\mu$M) | 53 | 180 | 350 | 530 | | IC50 |
| Protéines (%/Témoin non irradié) | -24% | -26 % | -25% | -27% | | |
| MPI (%/Témoin non irradié) | 118 % | 69 % | 17 % | -5 % | | |
| Effet anti-pigmentant du produit | -47% | -69% | -92% | -102% | | 0,37 mM vitamine C + 67 $\mu$M SMA |
| [SMA] ($\mu$M) | 53 | 180 | 350 | 530 | | IC50 |
| Protéines (%/Témoin non irradié) | -24% | -23% | -22 % | -20 % | | |
| MPI (%/Témoin non irradié) | 189 % | 126 % | 160 % | 166 % | | |
| Effet anti-pigmentant du produit | - | -44 % | -29 % | -26 % | | Non atteinte |

2.3. CONCLUSIONS

Aucune cyto-toxicité des produits testés n'a été observée aux doses évaluées. La vitamine C présente une activité anti-pigmentante et permet d'atteindre l'IC50 sur ce modèle à environ 0,47 mM. L'activité de la vitamine C à faibles concentrations sous UV est sensiblement améliorée en présence du copolymère SMA1000HNa®. En effet, l'IC50 de la vitamine C est atteinte à 0.37 mM en présence du copolymère SMA1000HNa®, soit plus rapidement que lorsqu'elle est testée seule. Cette légère amélioration pourrait être due au potentiel stabilisant du copolymère SMA1000HNa® sur la vitamine C, dans ces conditions expérimentales pro-oxydantes d'exposition aux rayons UV. Néanmoins, le copolymère SMA1000HNa® testé seul, dans la même gamme de concentration qu'en présence de vitamine C, présente également un potentiel anti-pigmentant qui lui est propre. Cet effet n'est pas synergique à celui de la vitamine C.

**Exemple 3 : mousse à raser**

| Ingrédients | % poids |
|---|---|
| Parfum | 0,60 |
| Triethanolamine | 2,90 |
| Butane | 1,00 |
| 2,4,4'-trichloro 2'-hydroxy diphényléther | 0,10 |
| Dimethiconol stearate (Mirasil wax S de chez Rhodia) | 1,00 |
| Hydroxyde de potassium | 0,40 |
| mono-éthanolamide d'acide de coprah oxypropyléné (2 OP) (PROMIDIUM CO de chez Uniqema) | 1,00 |
| Polyéthylèneglycol (14 000 OE) | 0,50 |
| Sodium laureth sulfate | 1,00 |
| Acide Myristique | 0,20 |
| Glycerine | 5,00 |
| acide palmitique | 3,80 |
| acide stéarique | 4,60 |
| Chlorhydrate de Pyridoxine (vitamine B6)/**actif anti-séborrhéique** | 0,04 |
| Isobutane | 2,38 |
| propane | 0,85 |
| Hydroxyethylcellulose | 0,28 |
| Conservateur | 0,95 |
| Copolymère styrene / acide maléique, sel de sodium à 40 % en poids dans l'eau * | 0,50 |
| Extrait de feuilles de menthe en solution aqueuse (Calmiskin® de chez Silab) **apaisant** | 0,50 |
| Huile de Tamanu **cicatrisant** | 0,10 |
| eau | qsp 100 |
| * : SMA1000HNa® commercialisé par la société CRAY VALLEY | |

Cette mousse à raser permet de traiter la séborrhée et prévient l'apparition d'hyperpigmentation pouvant apparaître pour une peau noire à tendance acnéique.

**Exemple 4 : crème traitante**

| Ingrédients | % poids |
|---|---|
| Polyacrylate de stéaryle (Intelimer IPA 13-1 de chez Landec) | 1,30 |
| sulfate de magnésium | 0,70 |
| Conservateur | 0,80 |

(suite)

| Ingrédients | % poids |
|---|---|
| Pidolate de zinc /**actif anti-séborrhéique** | 0,25 |
| micro-sphères de chlorure de vinylidene/acrylonitrile/polyméthylmétanhacrylate expancées à l'isobutane (Expancel 551 de chez Expancel) | 0,50 |
| Copolymère styrène / acide maléique, sel de sodium à 40 % en poids dans l'eau * | 1,00 |
| Mélange de stéarate d'éthylène glycol acétylé,tri-stéarate de glycéryle (Unitwix de chez United Guardian) | 0,50 |
| Isohexadecane | 7,75 |
| Cyclohexadecane | 8,70 |
| Glycérine | 7,00 |
| extrait de pétales de rose (Rose Flower Herbasol® extract de chez Cosmetochem) **anti irritant** | 1,00 |
| isononanoate d'isononyle | 7,75 |
| Sel disodique de l'acide diéthylène tétracétique | 0,04 |
| etyl diméthiconecopolyol(Abil EM 90 de Goldschmidt) | 3,80 |
| l'acide octadécènedioïque **desquamant** | 0,50 |
| Isostéarate de polyglycéryle-4 (Isolan GI 34 de Goldschmidt) | 1,25 |
| eau | qsp 100 |
| * : SMA1000HNa® commercialisé par la société CRAY VALLEY | |

Cette crème appliquée quotidiennement la nuit est utilisée pour le traitement des peaux grasses à tendance acnéique et évite l'hyperpigmentation pouvant apparaître sur les peaux noires.

**Exemple 5 : lotion traitante anti-acné**

| Ingrédients | % poids |
|---|---|
| Tocophérol | 0,05 |
| Bromure de myristyl triméthyl ammonium | 0,03 |
| Sel disodique de l'acide diéthylène tétracétique | 0,08 |
| Polyéthylène glycol (8 OE) | 5,0 |
| Glycérine | 3,0 |
| Huile de ricin hydrogénée oxyéthylénée (60 OE) | 0,5 |
| Copolymère styrene / acide maléique, sel de sodium à 40 % en poids dans l'eau * | 0,5 |
| Acide salicylique **desquamant** | 0,5 |
| Conservateurs | 0,8 |
| Parfum | 0,08 |
| Caprylylglycol **antiacné** | 0,2 |
| extrait de pétales de rose (Rose Flower Herbasol® extract de chez Cosmetochem) **anti irritant** | 1,0 |
| Glycine greffée sur chaîne undécylénique (Lipacide UG OR de chez Seppic) **anti séborrhéique** | 0,5 |
| Ethanol | 5,0 |

(suite)

| Ingrédients | % poids |
|---|---|
| eau | qsp 100 |
| *: SMA1000HNa® commercialisé par la société CRAY VALLEY | |

La lotion est appliquée sur les boutons d'acné d'une peau noire une fois par jour le soir. Elle permet la disparition des boutons sans former de taches d'hyperpigmentation.

**Revendications**

1. Composition comprenant dans un milieu physiologiquement acceptable au moins un copolymère de monomère styrénique et de diacide carboxylique à insaturation éthylénique et au moins un actif anti-séborrhéique.

2. Composition selon la revendication précédente, dans laquelle le monomère styrénique est choisi parmi le styrène et l'alpha-méthyl styrène.

3. Composition selon la revendication 1 ou 2, dans laquelle le monomère styrénique est le styrène.

4. Composition selon l'une quelconque des revendications précédentes, dans laquelle le diacide carboxylique à insaturation éthylénique est choisi parmi l'acide maléique, l'acide itaconique, et leurs dérivés anhydrides hydrolysés après polymérisation.

5. Composition selon l'une quelconque des revendications précédentes, dans laquelle le diacide carboxylique à insaturation éthylénique est choisi parmi l'acide maléique ou un de ses dérivés anhydrides hydrolysés après polymérisation.

6. Composition selon l'une quelconque des revendications précédentes, dans laquelle ledit copolymère est sous forme de sel.

7. Composition selon la revendication précédente, dans laquelle ledit copolymère est sous forme de sel choisi parmi les sels d'ammonium, de sodium, de potassium et de lithium, et de préférence sous forme de sel de sodium.

8. Composition selon l'une quelconque des revendications précédentes, dans laquelle ledit copolymère est un copolymère de styrène et d'acide maléique.

9. Composition selon l'une quelconque des revendications précédentes, dans laquelle l'actif anti-séborrhéique est choisi parmi l'acide rétinoïque ; le peroxyde de benzoyle ; le soufre ; la vitamine B6 ; le chlorure de sélénium ; la criste marine ; les mélanges d'extrait de canelle, de thé et d'octanoylglycine ; le mélange de canelle, de sarcosine et d'octanoylglycine ; les sels de zinc ; les dérivés de cuivre ; des extraits de végétaux des espèces *Arnica montana, Cinchona succirubra, Eugenia caryophyllata, Humulus lupulus, Hypericum perforatum, Mentha piperita, Rosmarinus officinalis, Salvia oficinalis* et *Thymus vulgaris ;* les extraits de reine des prés ; les extraits d'algue *laminaria saccharina ;* les mélanges d'extraits de racines de pimprenelle, de rhizomes de gingembre et d'écorce de cannelier ; les extraits de graines de lin ; les extraits de Phellodendron ; les mélanges d'huile d'argan, d'extrait de *serenoa serrulata* et d'extrait de graines de sésame ; les mélanges d'extraits d'epilobe, de *terminalia chebula,* de capucine et de zinc biodisponible ; les extraits de *Pygeum afrianum* ; les extraits de *serenoa serrulata* ; les mélanges d'extraits de plantain, de *berberis aquifolium* et de salicylate de sodium ; l'extrait de clou de girofle ; l'huile d'argan ; les filtrats de protéine lactique ; les extraits d'algue *laminaria ;* les oligosaccharides d'algue *laminaria digitata ;* les extraits de sucre de canne ; l'huile de schiste sulfonée ; les extraits d'ulmaire ; l'acide sébacique ; les glucomannanes extraits de tubercule de konjac et modifié par des chaînes alkylsulfonates ; les extraits de *Sophora angustifolia* ; les extraits de *cinchona succirubra* bark ; les extraits de *quillaja saponaria* ; la glycine greffée sur chaîne undécylénique ; le mélange d'acide oléanolique et d'acide nordihydroguaïarétique ; l'acide phthalimidoperoxyhexanoïque ; le citrate de trialkyle($C_{12}$-$C_{13}$); le citrate de trialkyle($C_{14}$-$C_{15}$) l'acide 10-hydroxydécanoïque ; les mélanges d'acide 10-hydroxydécanoïque, d'acide sébacique et de 1,10-décandiol et leurs mélanges.

**10.** Composition selon l'une quelconque des revendications précédentes, dans laquelle l'actif anti-séborrhéique est choisi parmi le peroxyde de benzoyle; la vitamine B6; les sels de zinc ; les extraits de reine des prés ; les extraits d'algue *laminaria saccharina;* les mélanges d'extraits de racines de pimprenelle, de rhizomes de gingembre et d'écorce de cannelier; l'extrait de clou de girofle; les filtrats de protéine lactique; les extraits d'ulmaire; l'acide sébacique; la glycine greffée sur chaîne undécylénique; le citrate de trialkyle($C_{12}$-$C_{13}$); le citrate de trialkyle($C_{14}$-$C_{15}$); l'acide 10-hydroxydécanoïque; les mélanges d'acide 10-hydroxydécanoïque, d'acide sébacique et de 1,10-décandiol et leurs mélanges.

**11.** Composition selon l'une quelconque des revendications précédentes, dans laquelle l'actif anti-séborrhéique est choisi parmi les sels de zinc; l'extrait de clou de girofle; la glycine greffée sur chaîne undécylénique; le citrate de trialkyle($C_{12}$-$C_{13}$); le citrate de trialkyle($C_{14}$-$C_{15}$) et leurs mélanges.

**12.** Composition selon l'une quelconque des revendications précédentes, dans laquelle l'actif anti-séborrhéique est présent dans une teneur allant de 0,1 à 10 % en poids, de préférence de 0,1 à 5 % en poids, et préférentiellement de 0,5 à 3 % en poids, par rapport au poids total de la composition.

**13.** Composition selon l'une quelconque des revendications précédentes, dans laquelle est en outre présent un actif anti-acné.

**14.** Composition selon l'une quelconque des revendications précédentes, dans laquelle l'actif anti-acné est un bactéricide éventuellement additionné d'un actif présentant des effets anti-adhésion bactérienne ou bien agissant sur le biofilm des bactéries pour éviter leur prolifération.

**15.** Composition selon la revendication précédente, dans laquelle le bactéricide est choisi parmi l'acide asiatique, le sel de monoéthanolamine du 1-hydroxy-4-methyl 6-trimethylpentyl 2-pyridone ; l'acide citronellique, l'acide périllique ; le 2-éthyl hexyle éther de glycérol ; le caprylate/caprate de glyceryle ; le phosphosilicate de calcium et de sodium ; les particules à base d'argent ; l'extrait de cône de houblon ; l'extrait de Millepertius ; le mélange d'extraits de racines de *scutellaria baicalensis,* de *paeonia suffruticosa* et de *glycyrrhiza glabra ;* l'extrait d' arganier ; les extraits de feuilles de busserole ; l'acide 10-hydroxy-2 décanoique, l'ursolate de sodium, l'acide azélaique, le di-iodo-méthyl p-tolylsulfone, la poudre de malachite, l'oxyde de zinc, l'acide octadécènedioïque ; l'acide ellagique ; le 2,4,4'-trichloro-2'-hydroxy diphényl éther, la 1-(3',4'-dichlorophényl)-3-(4'-chlorophényl)urée, le 3,4,4'-trichlorocarbanilide, le 3',4',5'-trichlorosalicylanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopirox, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octoxyglycérine, foctanoylglycine, le caprylyl glycol, l'acide 10-hydroxy-2-décanoïque, le dichlorophenyl imidazol dioxolan et ses dérivés, l'iodopropynyl butylcarbamate, le 3,7,11-triméthyldodéca-2,5,10-triénol, les phytosphingosines ; les sels d'ammonium quaternaires, les sels de cétylpyridinium, le cocoampho acétate de sodium, le diacetate disodium, les bétaines, le lauryl ether sulfate de sodium, le décyl glucoside, les malate de dialcools $C_{12-13}$ ramifiés, les monoesters de propylène glycol, le lauryl dimethylamine betaine, les polyammonium quaternaires et leurs mélanges.

**16.** Composition selon la revendication précédente, dans laquelle l'actif présentant des effets anti-adhésion bactérienne est choisi parmi le phytanetriol et ses dérivés ; les huiles végétales telles de l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia ; le cocoamphodiacétate disodique, le cocoate de glycéryle oxyéthyléné (7 OE), l'hexadécénylsuccinate 18, le palmitate d'octoxyglycéryle, le béhénate d'octoxyglycéryle, l'adipate de dioctyle, le PPG-15 stéaryl éther, le tartrate de di-alcools $C_{12}$-$C_{13}$ ramifiés et leurs mélanges.

**17.** Composition selon la revendication précédente, dans laquelle l'actif agissant sur le biofilm des bactéries pour éviter leur prolifération est choisi parmi le pentylène glycol, le nylon-66 ; l'huile de son de riz ; l'alcool polyvinylique; l'huile de colza ; les dérivés de fructose et leurs mélanges.

**18.** Composition selon l'une quelconque des revendications 13 à 17, **caractérisée en ce que** l'actif anti-acné est présent dans une teneur allant de 0,01 à 10 % en poids, de préférence de 0,05 à 5 % en poids par rapport au poids total de la composition.

**19.** Composition selon l'une quelconque des revendications précédentes, dans laquelle est en outre présent un agent desquamant.

**20.** Composition selon la revendication précédente, dans laquelle l'agent desquamant est choisi parmi les β-hydroxyacides; les α-hydroxyacides; l'acide 8-hexadécène 1,16-dicarboxylique ; l'acide gentisique et ses dérivés; les oligofucoses; l'acide cinnamique ; l'extrait de Saphora japonica; le resvératrol et certains dérivés d'acide jasmonique ; les composés aminosulfoniques; les dérivés de l'acide 2-oxothiazolidine-4-carboxylique ; les dérivés d'acides alpha aminés de type glycine ; le miel ; l'O-octanoyl-6-D-maltose et la N-acétyl glucosamine ; les oligofructoses, l'EDTA et ses dérivés, les laminaria extract, le o-linoleyl-6D-glucose, l'acide (3-hydroxy-2pentylcyclopentyl)-acétique, le trilactate de glycérol, l'O-octanyl-6'-D-maltose, la S carboxyméthyl cystéine, les dérivés siliciés de salicylate, les oligofucases, l'acide jasmonique et dérivés ; l'extrait de fleur de ficus *opuntia indica,* et leurs mélanges.

**21.** Composition selon l'une quelconque des revendications précédentes, dans laquelle est présent en outre au moins un agent apaisant et/ou au moins un actif astringent et/ou au moins un actif matifiant et/ou au moins un actif cicatrisant et/ou au moins un actif agissant sur la prolifération kératinocytaire.

**22.** Procédé cosmétique pour le traitement des peaux grasses et/ou de l'hyperséborrhée et pour la prévention des lésions acnéiques qui en découlent et/ou pour lutter contre les troubles esthétiques ou les imperfections associés à la surproduction de sébum pour les peaux foncées, notamment de phototype IV à VI, comprenant au moins une étape consistant à appliquer sur la peau, une composition telle que définie selon l'une quelconque des revendications précédentes.

**23.** Procédé cosmétique pour lutter conjointement contre les peaux grasses et contre l'hyperpigmentation localisée sur les sites de cicatrisation des lésions acnéiques découlant de l'hyperséborrhée de peaux foncées, notamment de phototype IV à VI, comprenant au moins une étape consistant à appliquer sur la peau, une composition telles que définies selon l'une quelconque des revendications 1 à 22.

**24.** Utilisation d'une association d'au moins un copolymère de monomère styrénique et de diacide carboxylique à insaturation éthylénique et d'au moins un actif anti-séborrhéique pour la préparation d'une composition dermatologique destinée à traiter les peaux grasses et/ou l'hyperséborrhée de peaux foncées, notamment de phototype IV à VI.

**Office européen**

**des brevets**

**RAPPORT DE RECHERCHE EUROPEENNE**

Numéro de la demande

EP 07 11 6048

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,A | FR 2 845 002 A1 (OREAL [FR]) 2 avril 2004 (2004-04-02) * page 3, ligne 28-31; revendications 1,7,8 * ----- | 1 | INV. A61K8/41 A61K8/81 A61Q19/00 A61Q19/02 |
| A | EP 1 374 853 A1 (OREAL [FR]) 2 janvier 2004 (2004-01-02) * alinéas [0014], [0015], [0028], [0029]; revendications 1,10,11 * ----- | 1 | |

DOMAINES TECHNIQUES RECHERCHES (IPC)

A61K

Le présent rapport a été établi pour toutes les revendications

| Lieu de la recherche | Date d'achèvement de la recherche | Examinateur |
|---|---|---|
| Munich | 7 janvier 2008 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...............................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**  EP 07 11 6048

La présente annexe indique les membres de la famille de brevets relatifs aux documents brevets cités dans le rapport de
recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-01-2008

| Document brevet cité au rapport de recherche | | Date de publication | Membre(s) de la famille de brevet(s) | | Date de publication |
|---|---|---|---|---|---|
| FR 2845002 | A1 | 02-04-2004 | AU | 2003278242 A1 | 19-04-2004 |
| | | | CN | 1684661 A | 19-10-2005 |
| | | | EP | 1558205 A2 | 03-08-2005 |
| | | | WO | 2004028483 A2 | 08-04-2004 |
| | | | JP | 2006504699 T | 09-02-2006 |
| EP 1374853 | A1 | 02-01-2004 | AT | 310497 T | 15-12-2005 |
| | | | AT | 320787 T | 15-04-2006 |
| | | | AT | 323533 T | 15-05-2006 |
| | | | AT | 307566 T | 15-11-2005 |
| | | | AT | 316778 T | 15-02-2006 |
| | | | CN | 1466939 A | 14-01-2004 |
| | | | CN | 1468594 A | 21-01-2004 |
| | | | DE | 60302009 D1 | 01-12-2005 |
| | | | DE | 60302009 T2 | 20-07-2006 |
| | | | DE | 60302389 D1 | 29-12-2005 |
| | | | DE | 60302389 T2 | 13-07-2006 |
| | | | DE | 60303429 T2 | 21-12-2006 |
| | | | DE | 60304133 T2 | 09-11-2006 |
| | | | DE | 60304626 T2 | 21-12-2006 |
| | | | EP | 1374849 A1 | 02-01-2004 |
| | | | EP | 1374850 A1 | 02-01-2004 |
| | | | EP | 1374851 A1 | 02-01-2004 |
| | | | EP | 1374852 A1 | 02-01-2004 |
| | | | ES | 2254885 T3 | 16-06-2006 |
| | | | ES | 2261888 T3 | 16-11-2006 |
| | | | ES | 2261889 T3 | 16-11-2006 |
| | | | ES | 2250850 T3 | 16-04-2006 |
| | | | ES | 2257652 T3 | 01-08-2006 |
| | | | JP | 3792213 B2 | 05-07-2006 |
| | | | JP | 2004035548 A | 05-02-2004 |
| | | | JP | 3950086 B2 | 25-07-2007 |
| | | | JP | 2004067675 A | 04-03-2004 |
| | | | JP | 3828510 B2 | 04-10-2006 |
| | | | JP | 2004026826 A | 29-01-2004 |
| | | | JP | 3795476 B2 | 12-07-2006 |
| | | | JP | 2004067676 A | 04-03-2004 |
| | | | JP | 3803657 B2 | 02-08-2006 |
| | | | JP | 2004067677 A | 04-03-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1374852 A
- WO 2004028483 A
- DE 10324567
- WO 9318743 A
- FR 0108283
- EP 1529523 A
- EP 1133979 A
- EP 1129694 A
- WO 0239975 A
- EP 0852949 A
- EP 0796861 A
- EP 0218200 A
- EP 1333022 A
- EP 1333021 A
- EP 761204 A
- EP 1562562 A
- EP 1151742 A
- FR 2869796
- US 5538793 A
- FR 2734825 A

**Littérature non-brevet citée dans la description**

- **R. SCHMIDT ; P. KRIEN ; M. RÉGNIER.** *Anal. Biochem.,* 1996, vol. 235 (2), 113-18